Europäisches Patentamt

European Patent Office

Office européen des brevets

(11) Publication number: **0 191 198**
**B1**

## (12) EUROPEAN PATENT SPECIFICATION

(45) Date of publication of patent specification: **12.07.89**

(21) Application number: **85201989.2**

(22) Date of filing: **27.11.85**

(51) Int. Cl.⁴: **A 45 D 33/00**, A 61 K 7/00

(54) Procedure for the production of a cosmetic article.

(30) Priority: **14.01.85 IT 1910385**

(43) Date of publication of application:
**20.08.86 Bulletin 86/34**

(45) Publication of the grant of the patent:
**12.07.89 Bulletin 89/28**

(84) Designated Contracting States:
**AT BE CH DE FR GB IT LI LU NL SE**

(56) References cited:
**EP-A-0 038 645**

(73) Proprietor: **INTERCOS ITALIANA S.p.A.**
**Via Buonarroti, 66**
**I-20064 Gorgonzola (Milano) (IT)**

(72) Inventor: **Avalle, Nadia**
**Via Panfilo Castaldi, 8**
**I-20124 Milano (IT)**

(74) Representative: **Mittler, Enrico et al**
**c/o Marchi & Mittler s.r.l. Viale Lombardia, 20**
**I-20131 Milano (IT)**

Courier Press, Leamington Spa, England.

## Description

The present invention relates to a procedure for the production of a cosmetic article.

Cosmetic products of various kinds such as face powders, eyeshadows and others are conventionally made by pressing cosmetic powder in metal moulds to secure a solid pastille which is then placed and secured in a special container.

A recent technique which is beneficial from the operative viewpoint as well as that of quality and appearance of the product obtained calls for the preparation of a fluid paste at 50—60°C containing cosmetic powder, fatty alcohol and a liquid support which is volatile at low temperature, and pouring it into a container with both a top and a bottom opening, which may be divided in compartments, appropriately arranged in an overturned position on a supporting surface acting as a mould. Cooling then brings on solidification of the paste and evaporation of the volatile support with temporary migration of the fatty alcohol toward the exposed surface to form a solidified pastille which adheres to the container and presents a top side shaped in conformance with the supporting surface. The bottom of the container is then closed and the cosmetic article formed by the cosmetic pastille and its container thus obtained can be returned to the erect position and inserted in the sales package for which it is designed. A technique of this kind is illustrated in US—A—4 337 859 and EP—A—0 038 645.

A drawback found in the aforesaid technique with pouring of the fluid paste is the tendency of the fluid paste to stick to the surface of the mould making detachment difficult and tending to give the cosmetic product an unacceptable visible side.

The object of the present invention is to accomplish a procedure for the production of a cosmetic article with the aforesaid technique using the pouring of fluid paste, being careful at the same time to avoid worsening of the appearance of the visible side as a result of the cosmetic product sticking to the mould surface.

According to the invention said object is achieved by a procedure comprising the preparation of a fluid paste containing cosmetic powder in a liquid component, the prearrangement of a container with an open top and a bottom having an opening overturned on a moulding surface, pouring of said fluid paste into said container through said bottom opening and subjecting the fluid paste to a heat treatment such as to bring about separation of the liquid ingredient of the paste and solidification of the paste to form a solid pastille with the top side shaped like the moulding surface, characterized in that between said moulding surface and said container top is inserted in advance a sheet of absorbant material.

The use of this sheet of absorbant material provides the important effect of allowing the liquid ingredient of the paste to filter through the pores of the absorbant material, abandoning the rest of the paste, which can solidify without sticking, assuming through the same absorbing material the conformation of the moulding surface. After appropriate drying the cosmetic product thus obtained detaches readily from the absorbant material, leaving a visible surface with a pleasing appearance.

The evaporation is thus facilitated with the obvious benefits of fast and efficient completion of the process.

The absorbant material also acts as a soft support for the cosmetic product, preventing the fluid paste from passing from one compartment to the other with the resulting mixing of products and colours if any when the container used has several compartments filled differently.

The absorbant material used can be of various kinds. In particular, it can consist of simple or cellulose or cellulose derivative paper or filter paper, natural or synthetic fiber fabric, nonwoven cloth and so on. Thickness can vary depending on the composition of the starting paste and the type of cosmetic product it is desired to obtain.

The fluid paste used can in turn be of any type in general use or, in accordance with another characteristic of the procedure according to the invention, it can consist of a cold oil-in-water emulsion in which the cosmetic powders are dispersed cold and which is poured cold into the container to then be subjected to heating to evaporate the aqueous ingredient of the paste and secure solidification of the pastille.

To prepare said emulsion for example a fatty ingredient consisting of a mixture of ethoxylated lanolin, glycerine, glyceryl monostearate, dimethicone, dioctyl sodium sulfosuccinate, various preservatives and water can be used. Said fatty ingredient is emulsified in cold water and to the emulsion thus formed is added, again under cold conditions, a dry ingredient consisting of cosmetic powder, dyes, pearls, and fillers such as talc or others.

These and other characteristics of the invention will be made clear by the detailed description below of some practical embodiments of the procedure according to the invention illustrated as nonlimiting examples in the annexed drawings wherein:—

Fig. 1 shows a perspective view of a multicompartment container which can be used to obtain a cosmetic article of several colours according to the procedure of the present invention;

Fig. 2 shows said container in plan view from above;

Fig. 3 shows a vertical cross section of said container along a plane cut III—III of Fig. 2;

Fig. 4 shows an overall perspective view of said container in overturned position and of a mould with an interposed absorbant sheet during the pouring step of fluid pastes of different colours into the various compartments of the container;

Fig. 5 shows the same overall view as Fig. 4 after pouring of said fluid pastes;

Fig. 6 shows the same overall view as Fig. 5 in vertical cross-section as in Fig. 3;

Fig. 7 shows an overall perspective view as in Fig. 5 during a final step in the application of a fixing resin;

Fig. 8 shows the same overall view as Fig. 7 in vertical cross-section as in Fig. 6;

Fig. 9 shows a perspective view of the finished cosmetic article released from the mould and from the absorbant sheet and returned to the erect position;

Fig. 10 shows the situation of Fig. 6 when a single-compartment container is used in combination with a mould with a concave surface to obtain a cosmetic article of modified form;

Fig. 11 shows a perspective view of the cosmetic article obtained with the variant of Fig. 10;

Fig. 12 shows the situation of Fig. 10 when a mould with a grooved concavity is used to obtain a cosmetic article with a surface design.

Fig. 13 shows a perspective view of a cosmetic article obtained with the variant of Fig. 12;

Fig. 14 shows a top view of a possible insertion of the cosmetic article of Fig. 9 in a square package;

Figs. 15—17 show top views of other possibilities of insertion of the cosmetic articles of modified form in their respective packages of various forms; and

Fig. 18 shows an overall perspective view of a cosmetic article according to the invention inserted in a casket with a cover.

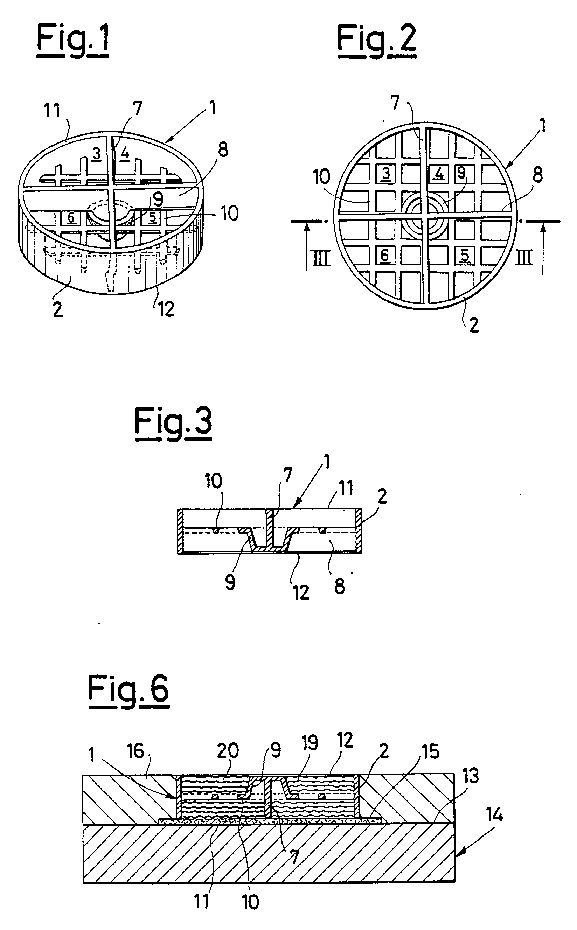

With reference to Figs. 1—3 a typical example of a multicompartment container usable with the procedure according to the invention for the formation of a cosmetic article of several colours, one per compartment, is shown therein and indicated by 1.

It comprises a cylindrical outer wall 2 which surrounds an internal space divided in four compartments 3, 4, 5 and 6 by a pair of diametrical partitions 7 and 8 perpendicular to each other which converge on a central truncated cone hub 9 developing from half the height of the container to the bottom thereof. A grate 10 is located at mid height inside said space and hence of the four compartments in which the space is divided.

The container 1 is shown in Figs. 1—3 in erect position, i.e. with the top 11 upward and the bottom 12 downward. As can be seen in Fig. 3 both the top and bottom of the container 1 open outward.

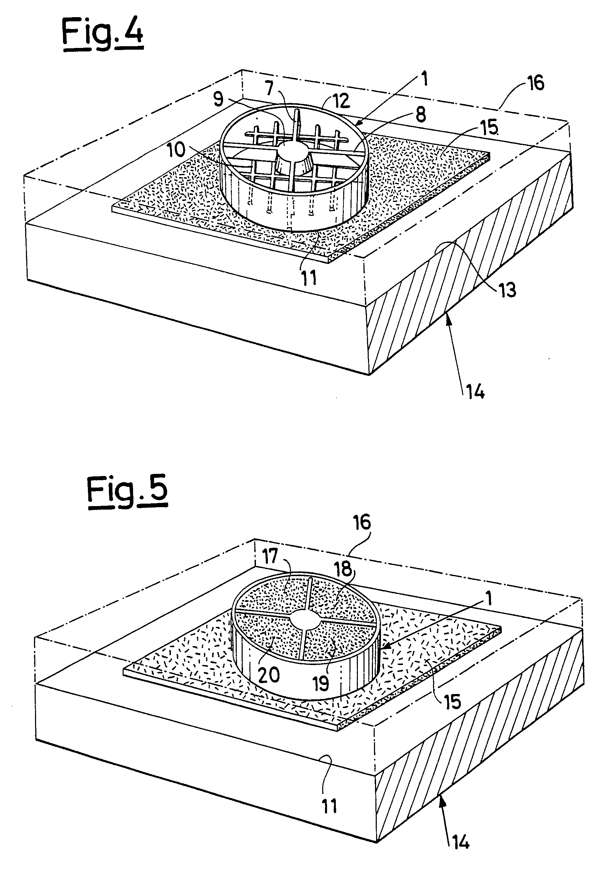

In accordance with the procedure according to the invention the container 1 is placed overturned on the moulding surface 13, for example flat as in Figs. 4—8, of a mould 14 with an interposed sheet of absorbant material 15, for example paper or cloth of appropriate thickness. A countermould 16 locks the container 1 in the desired position (Figs. 4—6).

Fluid pastes are prepared apart, each paste consisting of a cold oil-in-water emulsion in which are dispersed cold cosmetic powders of different colours. For example, according to the invention a fatty ingredient consisting of a mixture of ethoxylated lanolin, glycerin, glyceryl monostearate, dimethycone, dioctyl sodium sulfosuccinate, various preservatives and water is emulsified in cold water and to the emulsion thus formed is added, cold this time also, a dry ingredient consisting of cosmetic powders, dyes, pearls and fillers such as talc or others.

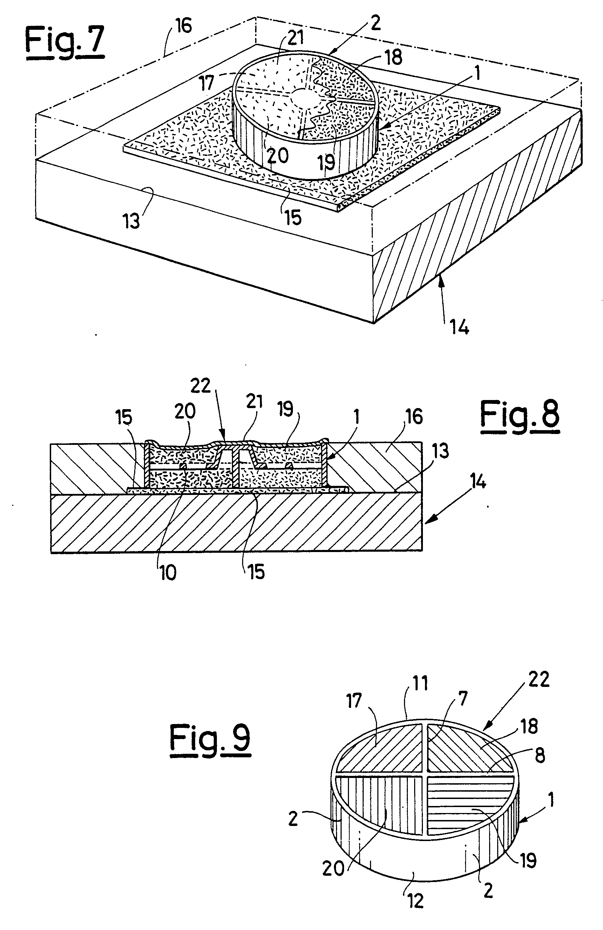

Each of said fluid pastes indicated with reference numbers 17, 18, 19 and 20 in the drawings, is then poured cold (Fig. 4) into one of the compartments 3, 4, 5 and 6 of the overturned container 1, through the openings in the bottom 12 thereof. The container 1 is thus filled with four distinct portions of fluid paste of different colours 17—20 separated by tha partitions 7 and 8 and by the seal provided by the absorbant sheet 15. This condition is shown in Figs. 5 and 6.

The filled container is left in the condition of Figs. 5 and 6 and subjected to a temperature of approximately 50°C for 10—12 hours to bring about drying and solidification of the various pastes. In this step the aqueous ingredient of the paste partly evaporates and partly filters through the absorbant sheet 15, thus abandoning the paste. As a result the solid pastilles 17—20 of slightly reduced height (Fig. 8) are formed, presenting on the bottom the smooth conformation of the moulding surface 13 and are firmly attached to the container 1 thanks to the grate 10 now incorporated in the pastille. A thin layer of fixing resin 21 is applied for greater safety to the bottom, which is still turned upward, of the container as shown in Figs. 7 and 8.

A finished cosmetic article 22 is thus obtained which, when extracted from the mould, returned to the erect position and freed of the absorbant sheet 15, appears as shown in Fig. 9, i.e. with four cosmetic pastilles 17—20 of different colours and of pleasing appearance housed in their respective compartments in container 1.

Alternatively, a container like that shown in Figs. 1—3 but without the partitions 7 and 8 and hence having a single compartment can be used for the procedure according to the invention with a single fluid paste to accomplish a cosmetic article like the one shown in Fig. 9 but with a single solid pastille of a single colour.

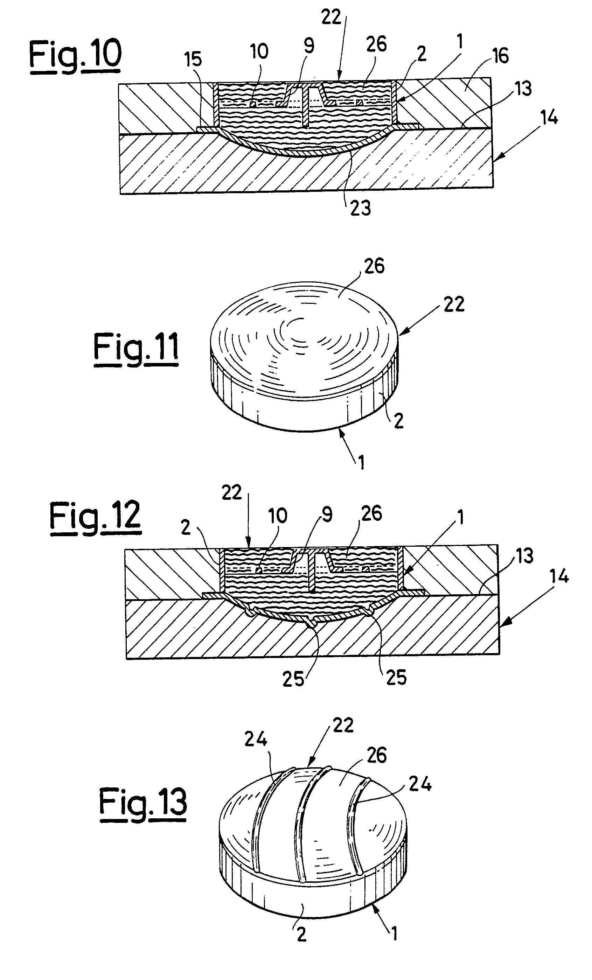

If desired, it is also possible to provide the cosmetic article with a convex instead of flat visible side as shown in Fig. 9. In this case a container 1 with a single compartment is placed in overturned position on a mold with its moulding surface provided with a concavity 23 (Fig. 10), again with the interposition of an absorbant sheet 15, which assumes in turn the concave form of the moulding surface. The result is a cosmetic article with a single pastille 26 with convex visible side such as illustrated in Fig. 11.

In addition to being convex the visible side of the cosmetic article can be designed with grooves 24 such as shown in Fig. 13. In this case the moulding surface must have the concavity 22 furnished with grooves 25 as shown in Fig. 12.

Other designs of the visible side of the cosmetic article can be accomplished by appropriately shaping the moulding surface.

In addition the cosmetic article can have a form

different from the circular form illustrated in Fig. 9, 11 and 13 just as it can be divided in compartments different from those illustrated in Fig. 9.

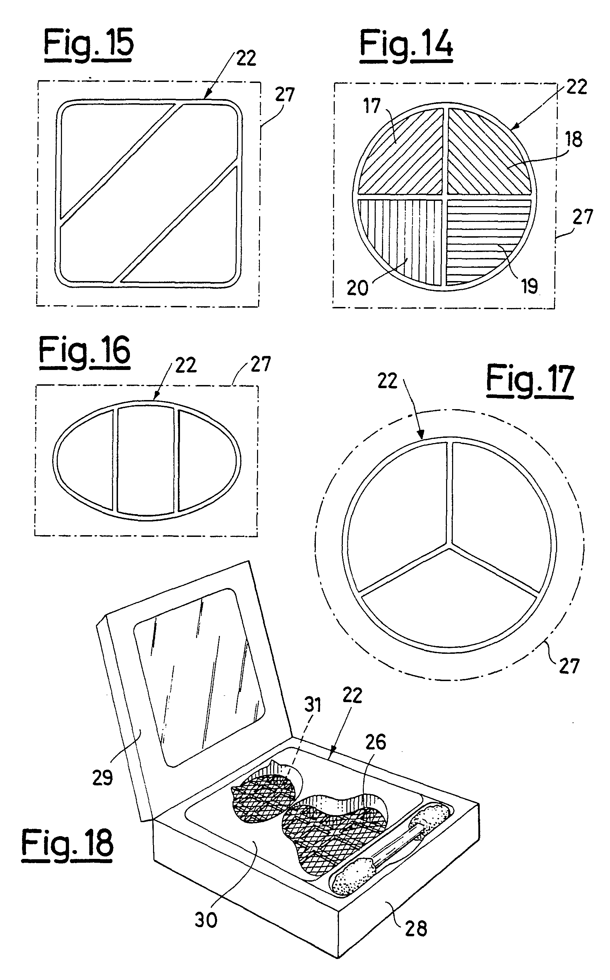

Whatever its conformation, the cosmetic article 22 is designed in general to be included in a sales package of a form corresponding to that of the article. Examples of sales packages are illustrated and indicated with reference number 27 in Figs. 14—17.

As an example, Fig. 18 shows a sales package consisting of a casket 28 with cover 29 in which is inserted a cosmetic article 22 including a shaped pastille 26, for example in the form of a cat, in a container 30 with a grate 31 furnished with a corresponding space to contain it.

## Claims

1. Procedure for the production of a cosmetic article comprising the preparation of a fluid paste (17—20) containing cosmetic powder in a liquid ingredient, the arrangement of a container (1) with open top (11), and a bottom (12) with an opening in overturned position on a moulding surface (14), the pouring of the fluid paste (17—20) into said container (1) through said bottom opening and the subjection of the fluid paste (17—20) to a heat treatment such as to bring about separation of the liquid ingredient from the paste and solidification of the paste itself until it forms a solid pastille (17—20) with the top side shaped like the moulding surface (14), characterized in that between said moulding surface (14) and said container top (11) a sheet (15) of absorbant material is first inserted.

2. Procedure according to claim 1 characterized in that said sheet (15) of absorbant material consists of absorbant paper.

3. Procedure according to claim 1 characterized by the fact that said sheet (15) of absorbant material consists of filter paper.

4. Procedure according to claim 1 characterized in that said sheet (15) of absorbant material consists of cloth.

5. Procedure according to claim 1 characterized in that said sheet (15) of absorbant material consist of nonwoven cloth.

6. Procedure according to claim 1 characterized in that said liquid ingredient is water.

7. Procedure according to claim 1 characterized in that said fluid paste (17—20) consists of a cold oil-in-water emulsion in which are dispersed cold cosmetic powders.

8. Procedure according to claim 7 characterized in that said fluid paste (17—20) comprises a fatty ingredient consisting of ethoxylated lanolin, glycerine, glyceryl monostearate, dimethycone, dioctyl sodium sulfosuccinate, various preservatives and water, and a dry ingredient consisting of cosmetic powder, dyes, pearls and various fillers, said fatty ingredient being emulsified in cold water and the dry ingredient being added cold to the emulsion thus formed.

9. Procedure according to claim 1 characterized in that said fluid paste (17—20) is poured cold into said container (1).

10. Procedure according to claim 9 characterized in that said heat treatment calls for heating the fluid paste (17—20) at a temperature higher than that at which it is poured.

11. Procedure according to claim 1 characterized in that it comprises the final application of a fixing resin (21) on the bottom side of the solidified pastille (17—20).

12. Procedure according to claim 1 characterized in that said container (1) has a grate (10) for anchoring the solidified pastille (17—20).

13. Procedure according to claim 1 characterized in that said container (1) is divided in compartments (3—6) to receive the respective fluid pastes.

14. Procedure according to claim 1, characterized in that said moulding surface (14) has a concavity (23) for the shaping of a solid pastille (17—20) with a convex top side.

15. Procedure according to claim 1 characterized in that said moulding surface (14) has designs (24—26) for shaping the top side of the solidified pastille (17—20).

## Patentansprüche

1. Verfahren zur Herstellung eines kosmetischen Artikels mit Vorbereiten einer einen kosmetischen Puder in einem flüssigen Bestandteil enthaltenden Fluidmasse (17—20), das Anordnen eines Behälters (1) mit offener Oberseite (11) und einem Boden (12) mit einer Öffnung in umgedrehter Stellung auf einer Formteiloberfläche (14), Gießen der Fluidmasse (17—20) in den Behälter (1) durch die Bodenöffnung und Aussetzen der Fluidmasse (17—20) einer Wärmebehandlung derart, daß die Trennung des flüssigen Bestandteiles von der Masse und Verfestigung der Masse selbst bewirkt wird, bis sie eine feste Pastille (17—20) bildet, deren Oberseite wie die Formteiloberfläche (14) geformt ist, dadurch gekennzeichnet, daß zwischen der Formteiloberfläche (14) und der Behälteroberseite (11) ein Blatt (15) eines absorbierenden Materiales zuerste eingefügt wird.

2. Verfahren nach Anspruch 1, dadurch gekennzeichnet, daß das Blatt (15) eines absorbierenden Materiales aus absorbierendem Papier besteht.

3. Verfahren nach Anspruch 1, gekennzeichnet durch die Tatsache, daß das Blatt (15) eines absorbierenden Materiales aus Filterpapier besteht.

4. Verfahren nach Anspruch 1, dadurch gekennzeichnet, daß das Blatt (15) eines absorbierenden Materiales aus Stoff besteht.

5. Verfahren nach Anspruch 1, dadurch gekennzeichnet, daß das Blatt (15) eines absorbierenden Materiales aus nicht-gewebtem Stoff besteht.

6. Verfahren nach Anspruch 1, dadurch gekennzeichnet, daß der flüssige Bestandteil Wasser ist.

7. Verfahren nach Anspruch 1, dadurch gekennzeichnet, daß die Fluidmasse (17—20) aus einer kalten Öl-in-Wasser-Emulsion besteht, in die kalt kosmetische Puder dispergiert sind.

8. Verfahren nach Anspruch 7, dadurch gekennzeichnet, daß die Fluidmasse (17—20) einen fetthaltigen Bestandteil, der aus äthoxyliertem Lanolin, Glyzerin, Glycerinmonostearat, Dimethykon, Dioktylnatriumsulfosuccinat, verschiedenen Schutzmitteln und Wasser besteht, und einen trockenen Bestandteil, der aus kosmetischem Puder, Farbstoffen, Perlen und verschiedenen Füllmitteln bestehet, aufweist, wobei der fetthaltige Bestandteil in kaltem Wasser emulgiert wird und der trockene Bestandteil kalt zu der so gebildeten Emulsion hinzugefügt wird.

9. Verfahren nach Anspruch 1, dadurch gekennzeichnet, daß die Fluidmasse (17—20) kalt in den Behälter (1) gegossen wird.

10. Verfahren nach Anspruch 9, dadurch gekennzeichnet, daß die Wärmebehandlung Erwärmen der Fluidmasse (17—20) auf eine Temperatur höher als die, bei der sie gegossen worden ist, verlangt.

11. Verfahren nach Anspruch 1, dadurch gekennzeichnet, daß es das Aufbringen zum Schluß eines Befestigungsharzes (21) auf die Bodenseite der festgewordenen Pastille (17—20) aufweist.

12. Verfahren nach Anspruch 1, dadurch gekennzeichnet, daß der Container (1) ein Gitter (10) zum Verankern der festgewordenen Pastille (17—20) aufweist.

13. Verfahren nach Anspruch 1, dadurch gekennzeichnet, daß der Container (1) in Fächer (3—6) unterteilt ist zum Aufnehmen der entsprechenden Fluidmaterialien.

14. Verfahren nach Anspruch 1, dadurch gekennzeichnet, daß die Formteiloberfläche (14) eine Rundhöhlung (23) aufweist zum Formen einer festen Pastille (17—20) mit einer konvexen Oberseite.

15. Verfahren nach Anspruch 1, dadurch gekennzeichnet, daß die Formteiloberfläche (14) Gestaltungen aufweist zum Formen der Oberseite der festgewordenen Pastille (17—20).

**Revendications**

1. Procédé pour la fabrication d'un article cosmétique comprenant la préparation d'une pâte fluid (17—20) contenant un cosmétique en poudre dans un ingrédient liquide, la constitution d'un récipient (1) dont le dessus (11) est ouvert et le fond (12) a une ouverture en position renversée sur une surface de moulage (14), le versement de la pâte fluide (17—20) dans ledit récipient (1) à travers ladite ouverture du fond, et la soumission de la pâte fluide (17—20) à un traitement thermique visant à provoquer la séparation de l'ingrédient liquide et de la pâte et la solidification de la pête elle-même jusqu'à ce qu'elle forme une pastille solide (17—20) dont la face supérieure à la même forme que la surface de moulage (14), caractérisé en ce qu'une feuille de matière absorbante (15) est d'abord intercalée entre ladite surface de moulage (14) et ledit dessus (11) du récipient.

2. Procédé selon la revendication 1, caractérisé en ce que ladite feuille (15) de matière absorbante est constituée de papier absorbant.

3. Procédé selon la revendication 1, caractérisé en ce que ladite feuille (15) de matière absorbante est constituée de papier filtre.

4. Procédé selon la revendication 1, caractérisé en ce que ladite feuille (15) de matière absorbante est constituée de textile.

5. Procédé selon la revendication 1, caractérisé en ce que ladite feuille (15) de matière absorbante est constituée de textile non tissé.

6. Procédé selon la revendication 1, caractérisé en ce que ledit ingrédient liquide est de l'eau.

7. Procédé selon la revendication 1, caractérisé en ce que ladite pâte fluide (17—20) est constituée d'une émulsion froide d'huile dans de l'eau dans laquelle sont dispersées des poudres cosmétiques froides.

8. Procédé selon la revendication 7, caractérisé en ce que ladite pâte fluide (17—20) comporte un ingrédient gras constitué de lanoline éthoxylée, de glycérine, de monostéarate glycéryle, de diméthycone, de dioctyl sodium sulfosuccinate, de divers stabilisants et d'eau, et un ingrédient sec constitué de poudre cosmétique, de colorants, de perles et diverses charges, ledit ingrédient gras étant émulsifié dans de l'eau froide et l'ingrédient sec étant ajouté à froid à l'émulsion ainsi formée.

9. Procédé selon la revendication 1, caractérisé en ce que ladite pâte fluide (17—20) est versée à froid dans ledit récipient (1).

10. Procédé selon la revendication 9, caractérisé en ce que ledit traitement thermique nécessite le chauffage de la pâte fluide (17—20) à une température supérieure à celle à laquelle elle est versée.

11. Procédé selon la revendication 1, caractérisé en ce qu'il comprend l'application finale d'une résine de fixation (21) sur la face inférieure de la pastille solidifiée (17—20).

12. Procédé selon la revendication 1, caractérisé en ce que ledit récipient (1) a une grille (10) pour fixer la pastille solidifiée (17—20).

13. Procédé selon la revendication 1, caractérisé en ce que ledit récipient (1) est divisé en compartiments (3—6) destinés à recevoir les pâtes fluides correspondantes.

14. Procédé selon la revendication 1, caractérisé en ce que ladite surface de moulage (14) a une concavité (23) pour la formation d'une pastille solide (17—20) à face supérieure convexe.

15. Procédé selon la revendication 1, caractérisé en ce que ladite surface de moulage (14) a des motifs (24—26) pour la mise en forme de la face supérieure de la pastille solidifiée (17—20).

# Fig.1

# Fig.2

# Fig.3

# Fig.6

1

# Fig.4

# Fig.5

Fig.7

Fig.8

Fig.9

Fig.10

Fig.11

Fig.12

Fig.13

## Fig.15

## Fig.14

## Fig.16

## Fig.17

## Fig.18